(19) Europäisches Patentamt
European Patent Office
Office européen des brevets

(11) EP 1 875 921 A1

(12) EUROPEAN PATENT APPLICATION
published in accordance with Art. 153(4) EPC

(43) Date of publication:
**09.01.2008 Bulletin 2008/02**

(21) Application number: **06745772.1**

(22) Date of filing: **27.04.2006**

(51) Int Cl.:
*A61K 38/00* (2006.01) *A61K 39/395* (2006.01)
*A61P 1/00* (2006.01) *A61P 3/00* (2006.01)
*A61P 3/10* (2006.01) *A61P 21/00* (2006.01)
*A61P 25/04* (2006.01) *A61P 25/14* (2006.01)
*A61P 25/16* (2006.01) *A61P 25/22* (2006.01)
*A61P 25/28* (2006.01) *A61P 29/00* (2006.01)

(86) International application number:
**PCT/JP2006/308844**

(87) International publication number:
**WO 2006/118196 (09.11.2006 Gazette 2006/45)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **27.04.2005 JP 2005130124**

(71) Applicant: **Ajinomoto Co., Inc.**
**Tokyo 104-8315 (JP)**

(72) Inventors:
- **IJICHI, Chiori**
  **Kawasaki-shi, Kanagawa 2108681 (JP)**
- **YAMADA, Naoyuki**
  **Kawasaki-shi, Kanagawa 2108681 (JP)**
- **HATANAKA, Toshihiro**
  **Kawasaki-shi, Kanagawa210 8681 (JP)**
- **TAKEHANA, Kenji**
  **Kawasaki-shi, Kanagawa 2108681 (JP)**
- **MIOTTO, Giovanni**
  **Padova 35121 (IT)**
- **MARIN, Oriano**
  **Padova 35121 (IT)**
- **CARPI, Andrea**
  **Padova 35121 (IT)**
- **BERTAGGIA, Denis**
  **Padova 35121 (IT)**

(74) Representative: **Nicholls, Kathryn Margaret et al**
**Mewburn Ellis LLP**
**York House**
**23 Kingsway**
**London WC2B 6HP (GB)**

# (54) DPP4 INHIBITOR AND PHARMACEUTICAL APPLICATION THEREOF

(57) The present invention provides a Dpp4 inhibitor which comprises a leucine derivative of the following formula (1) or a methionine derivative of the following formula (2):

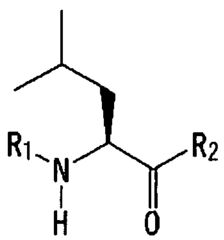
(1)

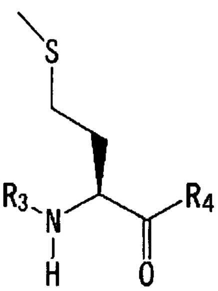
(2)

wherein each R1 and R3 represents a hydrogen atom (H) and an L-amino acid residue; R2 represents a hydroxyl group (OH), alkoxy group having 1 to 6 carbon atoms, amino group (NH2), alkylamino group having 1 to 6 carbon atoms, glycine residue, β-alanine residue, L-amino acid (except for proline, alanine and phenylalanine) residue or L-amino-acid amide (except for proline amide, alanine amide and phenylalanine amide) residue; and R4 represents a hydroxyl group

EP 1 875 921 A1

(OH), alkoxy group having 1 to 6 carbon atoms, amino group (NH2), alkylamino group having 1 to 6 carbon atoms, glycine residue, β-alanine residue, L-amino acid (except for proline and alanine) residue or L-amino-acid amide (except for proline amide and alanine amide) residue. These derivatives also act as autophagy regulators.

**Description**

Technical Field of the Invention

The present invention relates to Dpp4 (dipeptidyl peptidase-4) inhibitors and pharmaceutical use thereof; and autophagy regulators and pharmaceutical compositions which comprise them.

Background of the Invention

Autophagy is an ecological system in which cells decompose and reuse the self-components (organelle: cytoplasmic protein) because of the depletion of nutritional sources, and it is a nonspecific bulky protein decomposition in the cell. Autophagy is not only an acute body response that occurs when cells are subjected to the nutrient starvation stress but also a necessity to maintain homeostasis. Further, according to the recent studies, it has been clarified that autophagy relates to canceration, cell death, antigen presentation of immune cells, neurodegenerating diseases, cardiomyopathy, or the like. Especially, it is thought that the protein decomposition by autophagy or mitochondrial dysfunction deeply relates to the atrophy of organs such as muscles and digestive tracts caused by aging or debilitating diseases, or which is problematic after surgery (Non-patent Literature 1).

In the autophagy, new organelle called autophagosome is formed, and it covers organelle such as mitochondria and endoplasmic reticulum to encompass cytoplasmic components. Finally, it decomposes contents thereof in lysosome. Among the nutrients, amino acids are the best autophagy regulating factor. Particularly, leucine has the strongest autophagy inhibiting action (Non-patent Literature 2). Many prior studies are mainly on morphology and cytophysiology, and a large part of the regulatory mechanism of autophagy is not clear. The gene clusters required for autophagy are being identified by the gene analysis using an autophagy-defective strain in budding yeast, and it is clarified that many of such clusters are preserved in mammals. In the study using cultured hepatic cells, autophagy is induced by amino acid starvation or glucagon, and inhibited by amino acids or insulin (Non-patent Literature 3). However, it is hardly clarified in what mechanism amino acids are actually identified to cells in vivo and inhibit autophagy. Regarding the regulatory mechanism of autophagy by amino acids, it was disclosed by Giovanni Miotto, et al., who is one of the inventors of the present invention, that protein that expresses on the hepatocyte membrane (leucine receptor) takes an important role in inhibiting autophagy by leucine in the hepatic cells (Non-patent Literature 4). However, the molecular actual condition thereof has not been clarified.

Meanwhile, among amino acids, it is known that leucine has various physiologic and pharmacological actions in addition to the autophagy inhibiting action mentioned above. Among these, a variety of the actions are known such as the protein synthesis promoting action, endocrine hormone secretagogue action, glucose metabolism improving action, and appetite enhancing action (Non-patent Literature 5). However, the details have not been clarified in what mechanism in vivo these actions work. Since the membrane protein which inhibits autophagy (leucine receptor) that Miotto, et al. presumed on the hepatocyte membrane is thought to take an important role in these various physiologic functions of leucine, isolation and identification of such molecule has been expected.

Dpp4 is a membrane-bound peptide-decomposing enzyme which cleaves two residues from N terminal of various peptides. It is known that various peptide hormones in the blood become Dpp4 substrates and Dpp4 relates to the activation or inactivation thereof. Dpp4 exists in the free form in the blood, but the significance and free mechanism thereof are unknown. It is reported that the enzymatic activity of free Dpp4 in the blood increases or decrease in a certain type of mental diseases or inflammatory diseases such as hepatitis and inflammatory bowel disease. However, its relationship with the pathologies is not fully clarified. Hormones such as neuropeptides, immunopeptides, gut peptides and the like become Dpp4 substrates, and many of the second sequence of the N terminal thereof are proline or alanine. The most famous Dpp4 substrate is GLP-1 (glucagon-like peptide 1), and it is known that Dpp4 decomposes and inactivates GLP-1. Accordingly, Dpp4 inhibitors are expected to maintain the blood concentration of GLP-1 and promote physiologic insulin secretion from β-cells, and the study thereof is being developed as the target of drug discovery of diabetic agents. The second sequence of the N terminal of GLP-1 is serine, which is different from those of the other various substrates. It is known that Dpp4 expresses throughout the body, and it particularly numerously expresses in hepatic cells or epithelial cells of kidney, digestive tracts, skin or the like. The cell membrane-bound Dpp4 takes an important role in determining the cell polarity of the epithelial cells, and its activity as an adhesion factor is also presumed. Further, Dpp4 serves as a transfer factor of costimulatory signal in the T-lymph cell and it is important for activating CD4-positive T-cells (Non-patent Literature 6). The following URL describes a lot of information on the function and structure of human Dpp4 gene: http://www.ncbi.nlm.nih.gov/entrez/dispomim.cgi?id=102720

[Non-patent Literature 1] Science, 2004, 306: 990-995, Biochem. Biophys. Res. Com., 2004, 313: 453-458
[Non-patent Literature 2] Ann. Rev. Nutr. 1987, 7: 539-564
[Non-patent Literature 3] Science, 2004; 306: 990-995, Biochem. Biophys. Res. Com., 2004; 313: 453-458
[Non-patent Literature 4] J. Biol. Chem., 1994; 269: 25348-25353

[Non-patent Literature 5] Biochem. Biophys. Res. Com., 2004; 313: 387-458, Neurosci Lett. 2004; 354: 166-168
[Non-patent Literature 6] Clinical Science, 2000; 99: 93-104

Disclosure of the Invention

The object of the present invention is to provide novel Dpp4 inhibitors.
The further object of the present invention is to provide pharmaceutical compositions which comprise the above Dpp4 inhibitor(s).
The additional object of the present invention is to provide novel autophagy regulators.
The further additional object of the present invention is to provide pharmaceutical compositions, which comprise the above autophagy regulator(s).
The further additional object of the present invention is to provide preservatives of organs for transplantation, which comprise the above autophagy regulator(s).
The further additional object of the present invention is to provide drug screening methods.
In order to solve the above problems, the inventors attempted to identify leucine binding protein that exists on the membrane of rat hepatic cells by using a membrane-impermeable leucine derivative (Leu8-MAP). As a result of the thorough search, they found a molecular weight of 103-kilodalton leucine-specific binding protein (p 103) on the hepatocyte membrane. Though the bond of Leu8-MAP to p103 was inhibited by excess free leucine, it was not inhibited by isoleucine or valine. Therefore, p103 was protein that selectively bonds to leucine. Further, as a result of various examinations on the condition for purifying p103 from the hepatocyte membrane, they succeeded in isolating the above protein by purification and detected it as a single band protein by SDS-PAGE electrophoresis. This band was cleaved from the gel, and peptide fragmentation was conducted thereto by the in-gel reduction/alkylation and the enzyme digestion. Then, the mass spectrometry was conducted by providing the fragment with the equipment wherein a nanoHPLC system (Ultimate: Nippon Dionex K.K.) was connected to an ion trap mass spectrometer (LCQ: Thermo Electron Co., Ltd.). The obtained data was identified with a database search system (Mascot: Matrix Science, Ltd.). MSDB (Jan. 6, 2004 ver. by Matrix Science, Ltd.) was used as the sequence database for search. As a result, rat dipeptidyl peptidase IV (DPP4: EC 3.4.14.5) could be identified at a high score. From these results, the inventors ascertained that p103 was Dpp4 protein.
In addition to it, the inventors examined the effect of leucine on Dpp4 enzymatic activity by cell-free enzymatic assay. They also examined the relationship between Dpp4 on the hepatocyte membrane and autophagy in the hepatic cells by using the autophagy evaluation system with rat hepatic cells to complete the present invention.
Namely, the present invention provides a Dpp4 inhibitor which comprises a leucine derivative of the following formula (1) or a methionine derivative of the following formula (2):

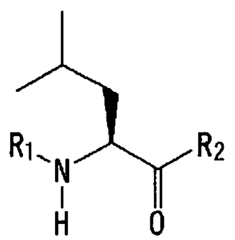 (1) 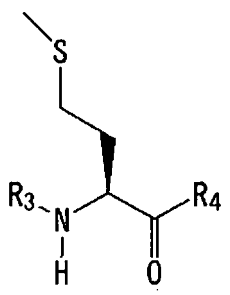 (2)

wherein each R1 and R3 represents a hydrogen atom (H) and an L-amino acid residue; R2 represents a hydroxyl group (OH), alkoxy group having 1 to 6 carbon atoms, amino group (NH2), alkylamino group having 1 to 6 carbon atoms, glycine residue, β-alanine residue, L-amino acid (except for proline, alanine and phenylalanine) residue or L-amino-acid amide (except for proline amide, alanine amide and phenylalanine amide) residue; and R4 represents a hydroxyl group (OH), alkoxy group having 1 to 6 carbon atoms, amino group (NH2), alkylamino group having 1 to 6 carbon atoms, glycine residue, β-alanine residue, L-amino acid (except for proline and alanine) residue or L-amino-acid amide (except for proline amide and alanine amide) residue.
The present invention also provides a Dpp4 inhibitor which comprises L-leucine and/or L-methionine.
The present invention further provides a therapeutic agent for diabetes, antiobesity agent, improving agent of drinking disorder, antianxiety agent or therapeutic agent for hypopathia, which comprises the above Dpp4 inhibitor(s).
The present invention additionally provides an autophagy regulator.
The present invention further additionally provides a pharmaceutical composition which comprises the autophagy regulator(s).

The present invention further additionally provides a preservative of organs for transplantation, which comprises the autophagy regulator(s).

The present invention further additionally provides a drug screening method in which the index is to bond to membrane Dpp4 or free Dpp4.

Brief Description of the Drawings

Figure 1 shows a diagram in which the concentration-dependent Dpp4 inhibiting activities are shown by being plotted regarding leucine, isoleucine, valine, 4-hydroxy-isoleucine and Diprotin A that is an existing Dpp4 inhibitor.

Figure 2 shows a diagram in which the result of the study on the effect of 20 kinds of amino acids that constitute protein on the Dpp4 enzymatic activity is described on the vertical axis as the substrate cleavage rate after the reaction.

Best Mode for Carrying out the Invention

Until now, certain types of dipeptide compounds are well known as Dpp4 inhibitors. For example, according to Biochem. J., 371, 525-532 (2003), after examining the substrate specificity of Dpp4 by using Xaa-Yaa-Aminomethyl Coumarine as an experimental substrate, it indicates that Xaa is identified as a substrate in a wide range of amino acids, while Yaa is specific to Pro and Ala. Namely, it is publicly known that peptides that have sequences of AA-Pro- or AA-Ala- become a good substrates specific to Dpp4.

In designing protease inhibitors or peptidase inhibitors, it is common to focus on peptides that have the partial structure of the substrate cleavage site. It is easy for those skilled in the art to presume that the peptides that have such sequence can have a competitive inhibiting activity. Actually, the Dpp4 inhibitors are well known wherein the proline part of dipeptide is structurally converted.

Further, Adv. Exp. Med. Biol., 421, 171-178 (1997) discloses the pKi value of Dpp4 inhibition of each compound such as AA-Pro, Ala-Ala, Ile-Ala and Leu-Phe. On the other hand, except for Leu-Pro, Leu-Ala, or Leu-Phe of which Ki value is indicated in the above reference, it is extremely difficult to detect from the substrate specificity of Dpp4 that dipeptides or derivatives which include Leu and the ester thereof, Leu-amide or Leu in the partial structure thereof widely have the Dpp4 inhibiting activity. Therefore, the Dpp4 inhibiting activity of the compounds of the present invention is a novel activity.

In the formulae (1) and (2) of the present invention, each R1 and R3 represents a hydrogen atom (H) and an L-amino acid residue. Examples of L-amino acid residues are those wherein a hydroxyl group is detached from a carboxyl group of an L-amino acid. Among them, the amino acid residue is preferably selected from the group consisting of L-Asp, L-Trp, L-Met, L-Asn, L-Tyr, L-Val, L-Arg, L-Orn, L-Leu, L-Phe, L-Gln and L-Ile.

Further, in the formulae (1) and (2) of the present invention, R2 represents a hydroxyl group (OH), alkoxy group having 1 to 6 carbon atoms, amino group (NH2), alkylamino group having 1 to 6 carbon atoms, glycine residue, β-alanine residue, L-amino acid (except for proline, alanine and phenylalanine) residue or L-amino-acid amide (except for proline amide, alanine amide and phenylalanine amide) residue; and R4 represents a hydroxyl group (OH), alkoxy group having 1 to 6 carbon atoms, amino group (NH2), alkylamino group having 1 to 6 carbon atoms, glycine residue, β-alanine residue, L-amino acid (except for proline and alanine) residue or L-amino-acid amide (except for proline amide and alanine amide) residue. Examples of L-amino acid residues and L-amino-acid amide residues are those wherein a hydrogen atom is detached from an amino group in the molecule thereof. Among them, it is preferable that each amino acid residues of R2 is selected from the group consisting of L-Val, L-Orn, L-Ile, L-Gln, L-Asp, L-Asn, L-Met, L-Lys, L-Thr and L-Ser. It is more preferable that it is selected from the group consisting of L-Val, L-Orn, L-Ile and L-Gln. It is also preferable that R2 is a hydroxyl group (OH). Meanwhile, the amino acid residues of R4 are preferably those other than L-Pro or L-Ala, and the same groups as those of R2 are also preferable.

In the formulae (1) and (2), it is preferable that either R1 or R2 is an amino acid residue, and that either R3 or R4 is an amino acid residue. In such a case, it is preferable that R1 and R3 are amino acid residues and R2 and R4 are hydroxyl groups (OH). It is also preferable that R1 and R3 are hydrogen atoms and R2 and R4 are amino acid residues.

The dipeptide derivatives or amide derivatives represented by the above formulae (1) and (2) can be obtained, for example, as mentioned in the following reaction scheme, by condensing leucine or methionine that protects an amino acid or a carboxyl group and suitable protected amino-acid derivatives or amines. As the peptide synthesis method, a number of excellent methods on the liquid-phase synthesis and the solid-phase synthesis are reported and either method may be used to synthesize, and it is not limited to a specific synthesis method.

Reaction Scheme

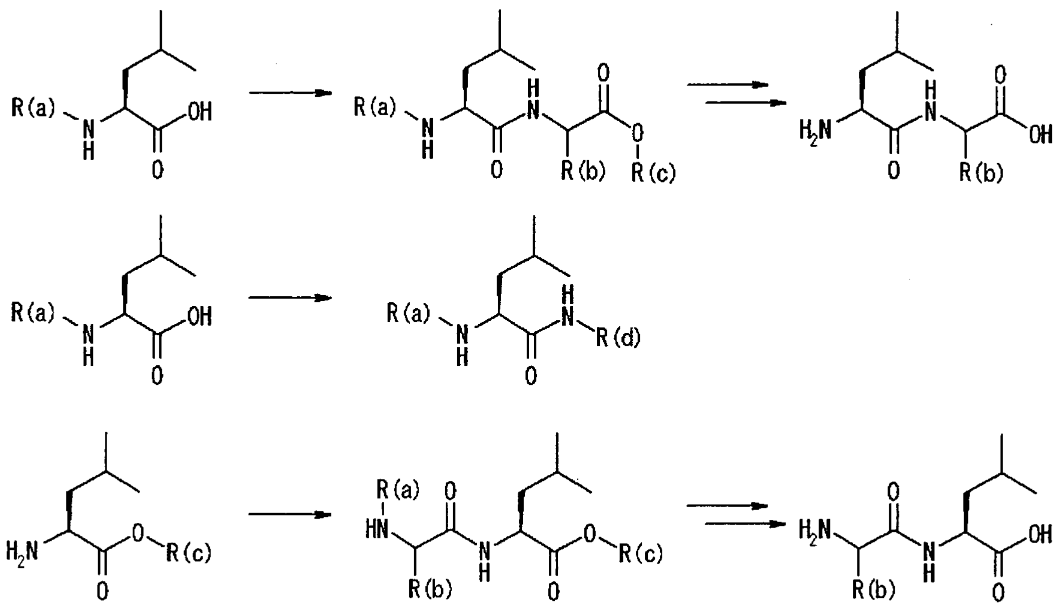

R(a): Fmoc, Boc, Cbz, etc.
R(b): amino acid side chain
R(c): Me, Et, Beznyl, etc.
R(d): H, alkyl group, etc.

Meanwhile, many of the compounds of the formulae (1) and (2) are available as reagents. In such a case, it is possible to purchase them for use.

The present invention also provides Dpp4 inhibitors which comprise L-leucine and/or L-methionine.

In the present invention, the above Dpp4 inhibitors can be used as an active ingredient of therapeutic agents for diabetes, antiobesity agents, improving agents of drinking disorder, antianxiety agents or therapeutic agents for hypopathia.

Namely, among the Dpp4 inhibitors of the present invention, the glucose metabolism improving effect of L-leucine is already known (JP-A 2003-171271, etc), and the use thereof in diabetes treatment or prevention is publicly known. Meanwhile, it is obvious that the compounds of the formula (1) of the present invention have the antidiabetic action from the fact that many of the Dpp4 inhibitors have the antidiabetic action because of the inhibiting activity thereof of the decomposition of GLP-1 (Diabetes 53: 2181-2189, 2004, Diabetes Care 26: 2929-2940, 2003).

Besides, it is known that animals which are genetically deficient in Dpp4 have behavioral features such as the decreased drinking behavior, the increased social activity and the increased sensitivity to pain (Physiology & Behavior 80 (2003) 123-134). Such knowledge indicates that the Dpp4 inhibitors of the present invention are useful as improving agents of drinking disorder, antianxiety agents or therapeutic agents for hypopathia.

The present invention further provides autophagy regulators which bond to Dpp4. Namely, the substances themselves that bond to Dpp4 are used as the autophagy regulators. Further, the present invention provides autophagy regulators which comprise the substances that bond to Dpp4 as an active ingredient. The preferable examples of the substances that bond to Dpp4 are Dpp4 antibodies. More specifically, they include monoclonal antibody clone 236.3 and monoclonal antibody OX-61. The monoclonal antibody clone 236.3 has the action of inhibiting autophagy, and the monoclonal antibody OX-61 has the action of promoting autophagy. These antibodies can be easily obtained as the marketed products.

The above autophagy regulators can be used as an active ingredient of pharmaceutical compositions. Examples of the pharmaceutical compositions are therapeutic or preventive agents of the symptoms such as the atrophy of intestine after surgery and the atrophy of organs such as muscles caused by chronic inflammations or cancer cachexia. In addition to them, the pharmaceutical compositions include therapeutic or preventive agents of various metabolic/endocrine disorders such as diabetes which are thought to be caused by accumulation of abnormal proteins, or neurodegenerating diseases such as Parkinson's disease, Alzheimer's disease and Huntington's disease.

The administered form of the pharmaceutical compositions of the present invention is not particularly limited.

The safe and necessary amount thereof can be parenterally or orally administered at once or via drip, and more specifically, intravenously, intra-arterially, subcutaneously, intramuscularly, or by infusion. Among them, the oral administration is preferable.

The pharmaceutical compositions of the present invention can be formulated into various dosage forms, e.g., in the case of oral agents, preparations such as tablets, capsules, granules, dispersants, troches, solutions, and subtle granules, or the preparations such as injection solvents, cream pharmaceuticals and suppositories. The preparation thereof can be conducted by publicly known methods. Both the active ingredient of the present invention and its preparation may contain pharmaceutically acceptable carriers, diluents, excipients, disintegrating agents, lubricants, flow improvers, or other necessary substances as the preparation, and the preparation can be produced by combination thereof, if necessary. Examples of the preparation carriers include lactose, glucose, D-mannitol, starch, crystalline cellulose, calcium carbonate, kaolin, starch, gelatin, hydroxypropyl cellulose, hydroxypropyl methyl cellulose, polyvinylpyrrolidone, ethanol, carboxy methyl cellulose, carboxy methyl cellulose calcium salts, magnesium stearate, talc, acetyl cellulose, sucrose, titanium oxide, benzoic acid, p-hydroxybenzoate ester, sodium dehydroacetate, gum arabic, tragacanth, methyl cellulose, egg yolk, surfactants, sucrose, simple syrup, citric acid, distilled water, ethanol, glycerin, propylene glycols, macrogol, sodium monohydrogen phosphate, sodium dihydrogen phosphate, sodium phosphate, glucose, sodium chloride, phenol, thimerosal, p-hydroxybenzoate ester and acid sodium sulfite. They are used by being mixed with the compounds of the present invention depending on the dosage forms.

The present invention further provides drug screening methods in which the index is to bond to membrane Dpp4 or free Dpp4. Among them, it is preferably the drug screening method in which the index is to bond to membrane and/or free Dpp4 by using the monoclonal antibody OX-61 that promotes autophagy and/or the monoclonal antibody clone 236.3 that inhibits autophagy.

As for the drug screening methods, for example, the screening of a substance that controls autophagy can be easily conducted by the method comprising the steps of using the monoclonal antibody OX-61 or the monoclonal antibody clone 236.3 wherein isotopes or fluorochromes are labeled by some physicochemical method; setting as the index the activity that changes the binding ability of cells that express membrane Dpp4 to the monoclonal antibody; and then screening the substance that modifies the binding ability from low-molecular-weight compound libraries or peptide libraries. At that time, the substance that promotes autophagy can be screened when using the monoclonal antibody OX-61, and the substance that inhibits autophagy can be screened when using the monoclonal antibody clone 236.3. Besides, recombinant Dpp4 may be used instead of the cells that express membrane Dpp4, and said recombinant Dpp4 which is prepared by being purified and isolated from the blood plasma of human beings or animals or by the recombinant DNA method.

Next, Examples will further illustrate the present invention. They only explain the present invention and do not particularly limit the invention.

## Examples

### Referential Example 1: Synthesis of Lys-Leu

#### 1) Synthesis of Leu-Wang resin

2.12g (6.01mmol) of Fmoc-L-leucine, 756mg (6.00mmol) of diisopropylcarbodiimide and 30mg (0.246mmol) of dimethylaminopyridine were dissolved in 20mL of DMF, and stirred with Wang resin (1.20mmol/g) in a 50mL syringe for the solid-phase synthesis for 3 hours at room temperature. After removing the solution, the resin was repeatedly washed six times with dimethylformamide, methanol and dichloromethane. 2mL of pyridine and 2mL of acetic anhydride were dissolved in 20mL of N-methyl pyrrolidone, syringed and then stirred for 2 hours at room temperature. After removing the solution, the resin was repeatedly washed six times with dimethylformamide, methanol and dichloromethane, and then dried in a vacuum pump. After 20mL of a solution of 20% piperidine/dimethylformamide was syringed and stirred for 10 minutes at room temperature, the solution was removed, and then the same procedure was repeated again. The resin was repeatedly washed six times with dimethylformamide, methanol and dichloromethane, and dried in a vacuum pump.

#### 2) Synthesis of ω-Boc-Lys-Leu- Wang resin

2.814g (6.00mmol) of Fmoc-(ω -Boc)-L-lysine, 940mg of diisopropylcarbodiimide and 30mg of dimethylaminopyridine were dissolved in 20mL of DMF, and stirred with the total amount of the above Leu-Wang resin in a 50mL syringe for the solid-phase synthesis overnight at room temperature. After removing the solution, the resin was repeatedly washed six times with dimethylformamide, methanol and dichloromethane. Then, 20mL of a solution of 20% piperidine/dimethylformamide was syringed, stirred for 10 minutes at room temperature, and the solution was removed. The same

procedure was repeated again. Then, the resin was repeatedly washed six times with dimethylformamide, methanol and dichloromethane, and dried in a vacuum pump.

3) Synthesis of Lys-Leu

100mg of the above w -Boc-Lys-Leu-Wang resin and 2mL of an aqueous solution of 95% trifluoroacetic acid were left (and occasionally stirred) in a 5mL syringe for the solid-phase synthesis for one hour at room temperature, and the solution was collected. The resin was washed with 2mL of an aqueous solution of 95% trifluoroacetic acid and 2mL of acetonitrile, respectively. The wash liquid and the reaction solution were mixed and concentrated under reduced pressure. The main product was isolated by the reverse phase HPLC and freeze-dried to obtain 4.5mg of Lys-Leu.
ESI-MS (m/z) [M+H]+ 260

The other dipeptides were synthesized by the same method as that of Referential Example 1.

Example 1: Inhibiting activity against rhDpp4

The inhibiting activity of each test compound was determined against the cleaving activity of GLY-PRO-p-nitroanilide (G-P-pNA, by Sigma, #G-0513), which is a synthetic substrate of recombinant human dipeptidyl peptidase IV (rhDpp4, by R&D Systems, #1180-SE). G-P-pNA was cleaved between PRO and pNA by rhDpp4. Since pNA is absorbed at 405nm, the value thereof was measured to determine the cleavage amount.

The reaction solution (25mM Tris (pH8.0), 100uM G-P-pNA, 0.25ng/ul rhDPP4) was prepared and added to the test compound so that the final concentration of the test compound became 2mM (1% DMSO concentration). The reaction solution was kept in 37 °C for 30 minutes. After that, the absorbance at 405nm was measured at once by a spectrophotometer. The reaction was conducted in 200ul scale with a 96-well microplate.

At the same time, the absorbance at 405nm of each 100, 33, 11, 3.7, 1.2, 0.41, and 0.14uM pNA solution was measured to obtain the relational formula of pNA concentration and absorbance, and the pNA amount (the substrate cleavage amount) in each reaction solution was calculated.

As for the inhibition rate, 3 to 4 wells without the test compound were prepared in the same plate. Then, the inhibition rate was calculated and shown in percentage by using the following formula from the proportion of the substrate cleavage amount (B) of the well to which each test compound was added to the average amount (A) of the substrate cleavage amount of the well without the test compound.

$$\text{Inhibition rate (\%)} = 100 \times (1 - (B)/(A))$$

In Figure 1, the concentration-dependent Dpp4 inhibiting activities are shown by being plotted regarding leucine, isoleucine, valine and 4-hydroxy-isoleucine among 20 kinds of amino acids that constitute protein, and Diprotin A that is an existing Dpp4 inhibitor. From these results, it is obvious that leucine inhibits Dpp4. In Figure 2, the result of the study on the effect of 20 kinds of amino acids that constitute protein on the Dpp4 enzymatic activity is shown on the vertical axis as the substrate cleavage rate after the reaction. From Figure 2, it is found that Met as well as Leu has the Dpp4 inhibiting activity among 20 kinds of the amino acids.

Meanwhile, Table 1 collectively indicates the result of the Dpp4 inhibiting activities of example compounds of the formula (1) calculated by the above method.

Table 1

| R1 | R2 | Inhibition rate (%) |
|---|---|---|
| H | NH2 | 33.2 |
| | NHC2H5 | 35.0 |
| | OCH3 | 31.1 |
| L-Asp | | 91.1 |
| L-Trp | | 89.0 |
| L-Met | | 88.2 |
| L-Asn | | 87.3 |

(continued)

| R1 | R2 | Inhibition rate (%) |
|---|---|---|
| L-Tyr | OH | 87.3 |
| L-Val | | 83.8 |
| L-A rg | | 83.7 |
| L-Cys | | 81.8 |
| L-Leu | | 80.0 |
| L-Phe | | 71.2 |
| L-Gln | | 63.8 |
| L-Ile | | 51.4 |
| L-Glu | | 49.0 |
| L-Ser | | 47.9 |
| L-His | | 46.9 |
| L-Lys | | 38.4 |
| L-Orn | | 32.0 |
| L-Ala | | 23.4 |
| L-Thr | | 18.2 |
| H | L-Val | 68.1 |
| | L-Orn | 61.2 |
| | L-Ile | 51.0 |
| | L-Gln | 50.5 |
| | L-Asp | 46.6 |
| | L-Asn | 44.3 |
| | L-Met | 43.2 |
| | L-Lys | 42.7 |
| | L-Thr | 42.0 |
| | L-Ser | 41.2 |
| | L-Phe | 40.4 |
| | L-His | 38.9 |
| | β Ala | 35.8 |
| | Gly | 33.2 |

Example 2: Measurement of autophagy by using rat hepatic cells

Male Wistar rats of 140 to 180g which were freely fed were used to prepare rat hepatic cells by the collagenase perfusion method (Seglen, P.O. Preparation of isolated liver cells. (1976) In Methods in Cell Biology eds. D.M. Prescott, 13, 29-83. Academic: New York / London). By this method, 2 to 2.5 x 108 cells of the hepatic cells per one individual's kidney could be prepared.

The protein decomposition (autophagy) of the prepared hepatic cells was determined by the method of Venerando, et al. Namely, the hepatic cells were put in a 10mL flask with 3.5mL of KRB buffer (24mM bicarbonate, 6mM glucose) to prepare a cell suspension (1 to 1.2 x 106 cells/mL). The solution was kept and left in 37 °C for 45 minutes in a carbon dioxide incubator. Then, various chemical treatments were conducted under the existence of 20uM cycloheximide in order to stop the protein synthesis, and the solution was kept at 37°Cfor 30 minutes. At each 31 minutes and 42 minutes, 0.35mL of the hepatic cell suspension was taken out, and a perchloric acid cooled with ice (final concentration

6%) was added to the latter and preserved at -20°C. Valine in the supernatant of the acid-soluble fraction was measured, and valine release for 11 minutes was determined.

The experiment was independently conducted three times (Venerando, R., Miotto, G., Kadowaki, M., Siliprandi, N., and Mortimore, G.E. (1994) Multiphasic control of proteolysis by leucine and alanine in the isolated rat hepatocytes. Am. J. Physiol. 266, C455-C461). The determination of the amino acids was conducted by the method of Tapuhi, et al. (Tapuhi, Y, Schmidt, D., Lindner, W., and Karger, B.L. (1981) Dansylation of amino acids for high-performance liquid chromatography, Anal. Biochem. 115, 123-129).

The materials for experiment are as follows: the monoclonal antibodies against Dpp4: MRC OX61 isotype IgG2a (Oxford Biotechnology), and clone 236.3 isotype IgG2bk (Pierce Endogen).

Table 2 shows the effects of leucine and the Dpp4 antibodies against the autophagy induced to rat hepatic cells by amino acid starvation.

Table 2

TABLE: *Effect of DPP TV modulators on inhibition of starvation induced proteolysis*

| | | Inhibition of proteolysis (%) | St. Dev. | N. of Experiments |
|---|---|---|---|---|
| A | No Addition | **0** | 8.9 | 13 |
| B | Leucine | **27.9** | 11.6 | 13 |
| C | Leu + OX-61 | **38.1** | 11.5 | 6 |
| D | clone OX-61 | **-9.9** | 6.4 | 6 |
| E | clone 236.3 | **12.3** | 12.6 | 7 |

It is obvious that leucine strongly inhibits autophagy induced by amino acid starvation as is well known until now (Ann. Rev. Nutr. 1987, 7: 539-564). On the other hand, the effects of the Dpp4 antibodies against autophagy are different depending on the clone. In case of a separate treatment, OX-61 promotes autophagy and 236.3 inhibits autophagy. It is clarified that, when OX-61 is added with leucine, it further enhances the autophagy inhibiting activity by leucine. These results indicate that a substance that bonds to Dpp4 can control autophagy activity induced by amino acid starvation. Therefore, it indicates that autophagy activity can be controlled by the substance that bonds to Dpp4, such as the antibodies against Dpp4 or existing Dpp4 inhibitors.

As the substances which control the autophagy activity other than amino acids, it is known that insulin inhibits autophagy and glucagon promotes autophagy. Meanwhile, as the example of low-molecular compounds, though it is reported that vitamin C has the effect of inhibiting the autophagy of glial cells (Journal of Neurochemistry, 2002, 82, 538-549), the compounds other than vitamin C are hardly known. Further, it is thought that Dpp4 controls the nutritional metabolism in vivo by controlling the activity of various peptide hormones (Clinical Science, 2000; 99: 93-104). However, it is not known that Dpp4 relates to cell death or the atrophy of organs due to autophagy accompanying the nutrient starvation.

Autophagy is a normal body response induced by the nutrient starvation and an important mechanism to maintain homeostasis in which the body in its own reuses amino acids that are necessary in growing cells or the normal cell function. However, it has been also known that the atrophy of organs due to the excess nutrient starvation or the disruption of the mechanism variously abnormalizes organs and becomes the cause of the diseases. Under the circumstances, controlling autophagy induced by the nutrient starvation by substances other than amino acids is an effective method for preventing/treating various diseases that are caused by the abnormal autophagy or the atrophy of organs due to malnutrition or inflammations. Examples of the atrophy of organs are the atrophy of digestive tracts because of no intake of foods after surgery and the atrophy of organs such as muscles because of chronic inflammations or cancer cachexia. Examples of the diseases caused by the abnormal autophagy are various metabolic/endocrine disorders such as diabetes which are thought to be caused by accumulation of abnormal proteins, and neurodegenerating diseases such as Parkinson's disease, Alzheimer's disease and Huntington's disease (Science, 2004; 306: 990-995, Biochem. Biophys. Res. Com., 2004; 313: 453-458). Further, it is known that autophagy occurs in the preserved organs for transplantation or in the organs in the reperfusion after a transplantation (Arch Histol Cytol, 68(1): 71-80 (2005)).

## Claims

1. A Dpp4 inhibitor which comprises a leucine derivative of the following formula (1) or a methionine derivative of the following formula (2):

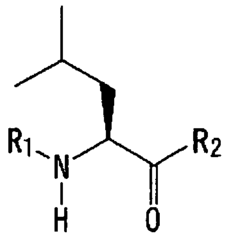 (1) 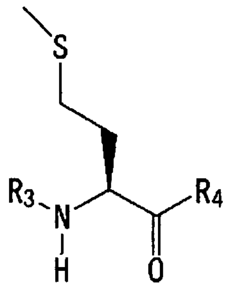 (2)

wherein each R1 and R3 represents a hydrogen atom (H) and an L-amino acid residue; R2 represents a hydroxyl group (OH), alkoxy group having 1 to 6 carbon atoms, amino group (NH2), alkylamino group having 1 to 6 carbon atoms, glycine residue, β-alanine residue, L-amino acid (except for proline, alanine and phenylalanine) residue or L-amino-acid amide (except for proline amide, alanine amide and phenylalanine amide) residue; and R4 represents a hydroxyl group (OH), alkoxy group having 1 to 6 carbon atoms, amino group (NH2), alkylamino group having 1 to 6 carbon atoms, glycine residue, β-alanine residue, L-amino acid (except for proline and alanine) residue or L-amino-acid amide (except for proline amide and alanine amide) residue.

**2.** The Dpp4 inhibitor according to claim 1, wherein, in the formula (1), the amino acid residue of R2 is selected from the group consisting of L-Val, L-Orn, L-Ile, L-Gln, L-Asp, L-Asn, L-Met, L-Lys, L-Thr, and L-Ser.

**3.** A Dpp4 inhibitor which comprises L-leucine and/or L-methionine.

**4.** A therapeutic agent for diabetes, antiobesity agent, improving agent of drinking disorder, antianxiety agent or therapeutic agent for hypopathia, which comprises the Dpp4 inhibitor(s) according to any one of claims 1 to 4.

**5.** An autophagy regulator which bonds to Dpp4.

**6.** The autophagy regulator according to claim 5, which comprises a Dpp4 antibody(ies).

**7.** The autophagy regulator according to claim 6, wherein the Dpp4 antibody is monoclonal antibody clone 236.3.

**8.** The autophagy regulator according to claim 6, wherein the Dpp4 antibody is monoclonal antibody OX-61.

**9.** A pharmaceutical composition which comprises the autophagy regulator(s) according to any one of claims 5 to 8.

**10.** The pharmaceutical composition according to claim 9, which is a therapeutic or preventive agent of the atrophy of intestine after surgery or due to malnutrition or aging; or said therapeutic or preventive agent of the symptoms caused by chronic inflammations or cancer cachexia.

**11.** The pharmaceutical composition according to claim 9, which is a therapeutic or preventive agent of metabolic/endocrine disorders or neurodegenerating diseases.

**12.** A preservative of organs for transplantation, which comprises the autophagy regulator(s) according to any one of claims 5 to 8.

**13.** A drug screening method in which the index is to bond to membrane Dpp4 or free Dpp4.

**14.** The drug screening method according to claim 13, in which the index is to bond to the membrane and/or free Dpp4 by using monoclonal antibody OX-61 that promotes autophagy and/or monoclonal antibody clone 236.3 that inhibits autophagy.

FIG.1

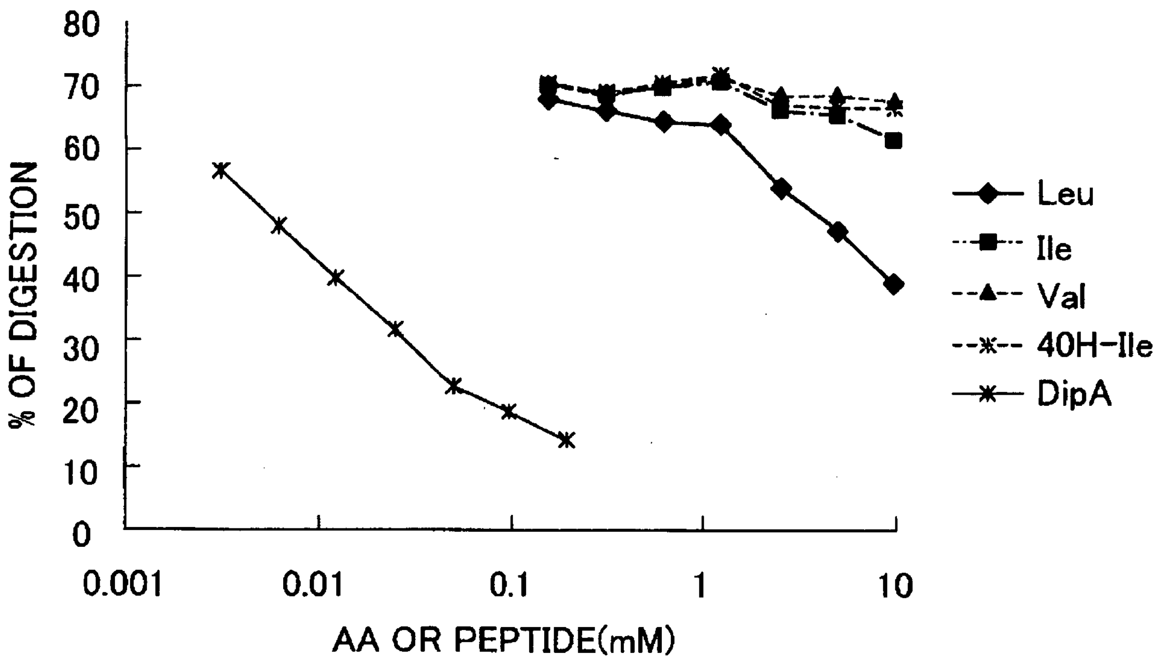
80
70
60
50
40
30
20
10
0
% OF DIGESTION
0.001
0.01
0.1
1
10
AA OR PEPTIDE(mM)
Leu
Ile
Val
4OH-Ile
DipA

FIG.2

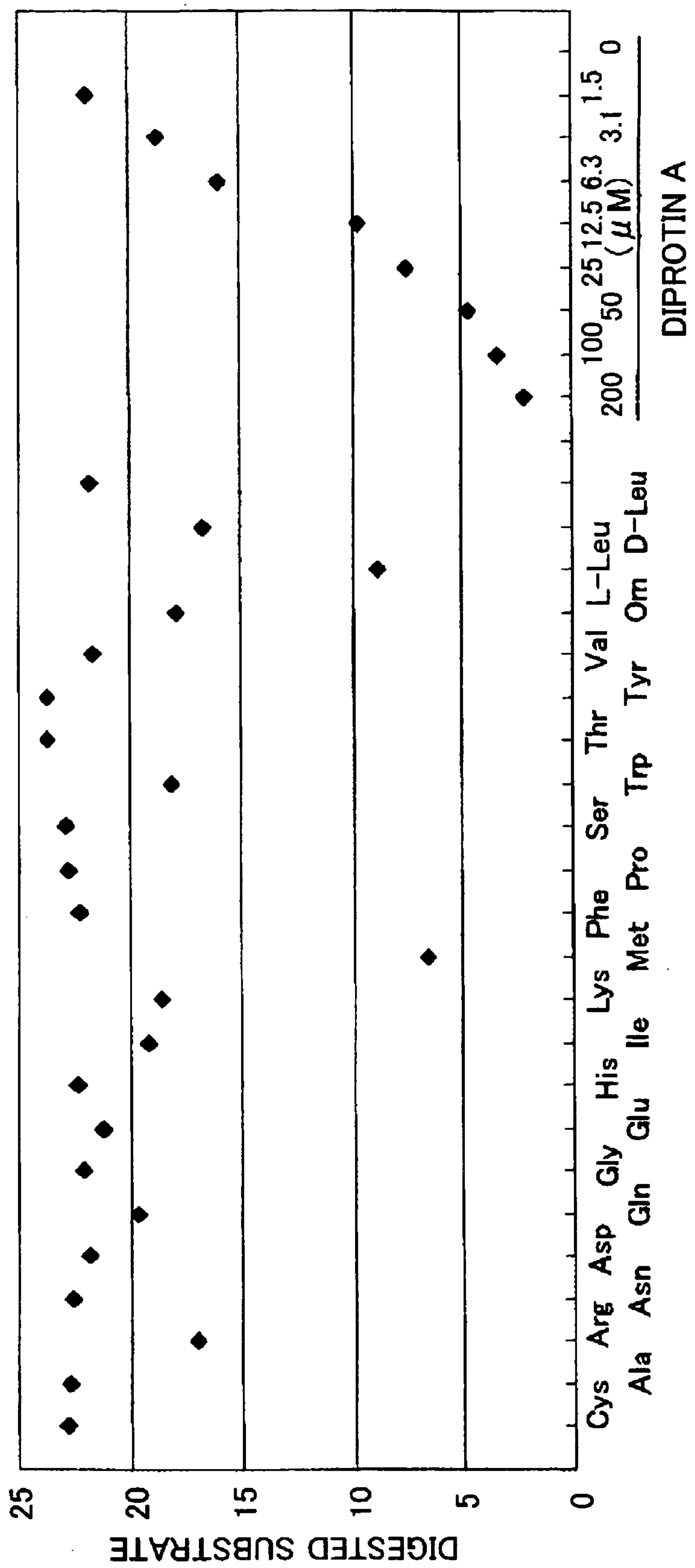
DIGESTED SUBSTRATE
0
5
10
15
20
25
Cys
Ala
Arg
Asn
Asp
Gln
Gly
Glu
His
Ile
Lys
Met
Phe
Pro
Ser
Trp
Thr
Tyr
Val
Orn
L-Leu
D-Leu
200
100
50
25
12.5
6.3
3.1
1.5
0
(μM)
DIPROTIN A

**INTERNATIONAL SEARCH REPORT**

International application No.
PCT/JP2006/308844

A. CLASSIFICATION OF SUBJECT MATTER
***A61K38/00***(2006.01), ***A61K39/395***(2006.01), ***A61P1/00***(2006.01), ***A61P3/00***(2006.01), ***A61P3/10***(2006.01), ***A61P21/00***(2006.01), ***A61P25/04***(2006.01), ***A61P25/14***(2006.01), ***A61P25/16***(2006.01), ***A61P25/22***(2006.01),
According to International Patent Classification (IPC) or to both national classification and IPC

B. FIELDS SEARCHED

Minimum documentation searched (classification system followed by classification symbols)
A61K38/00, A61K39/395, A61P1/00, A61P3/00, A61P3/10, A61P21/00, A61P25/04, A61P25/14, A61P25/16, A61P25/22, A61P25/28, A61P29/00, A61P35/00, A61P43/00, C12N9/99, G01N33/15, G01N33/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), CAplus(STN), EMBASE(STN), MEDLINE(STN), JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | JP 2003-171271 A (Ajinomoto Co., Inc.), 17 June, 2003 (17.06.03), Claim 1 (Family: none) | 1-4 |
| Y | WRENGER Sabine, EFFECTS OF NONAPEPTIDES DERIVED FROM THE N-TERMINAL STRUCTURE OF HUMAN IMMUNODEFICIENCY VIRUS-1(HIV-1)TAT ON SUPPRESSION OF CD26-DEPENDENT T CELL GROWTH, Advances in experimental medicine and biology, 2000, Vol.477, pages 161 to 165 | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>07 June, 2006 (07.06.06) | Date of mailing of the international search report<br>20 June, 2006 (20.06.06) |
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/308844

C (Continuation). DOCUMENTS CONSIDERED TO BE RELEVANT

| Category* | Citation of document, with indication, where appropriate, of the relevant passages | Relevant to claim No. |
|---|---|---|
| Y | WEIHOFEN Wilhelm Andreas, Crystal Structures of HIV-1 Tat-derived Nonapeptides Tat-(1-9) and Trp2-Tat-(1-9) Bound to the Active Site of Dipeptidyl-peptidase IV(CD)26, Journal of Biological Chemistry, 28 January, 2005 (28.01.05), Vol.280, No.15, pages 14911 to 14917 | 1-4 |
| X<br>A | JP 10-182613 A (Tanabe Seiyaku Co., Ltd.), 07 July, 1998 (07.07.98), Claim 1<br>& WO 98/18763 A1 | 13-14<br>1-4 |
| X<br>A | WO 2004/26822 A2 (ABBOT LABORATORIES), 01 April, 2004 (01.04.04), Claim 1<br>& EP 1560811 A2 & US 2004/121964 A1 | 13-14<br>1-4 |
| A | NISHITANI Shinobu, Pharmacological activities of branched-chain amino acids: specificity of tissue and signal transduction, Biochemical and Biophysical Research Communications, 2004, Vol.313, pages 387 to 389 | 1-4 |
| A | MENTLEIN Rolf, Dipeptidyl-peptidase IV hydrolyses gastric inhibitory polypeptide, glucagon-like peptide-1(7-36)amide, peptide histidine methionine and is responsible for their degradation in humna serum, Eur.J. Biochem., 1993, Vol.214, pages 829 to 835 | 1-4 |
| A | SHINTANI Takahiro, Autophagy in Health and Disease: A Double-Edged Sword, SCIENCE, 2004, Vol.306, pages 990 to 995 | 5-12 |
| A | YOSHIMORI Tamotsu, Autophagy: a regulated bulk degradation process inside cells, Biochemical and Biophysical Research Communications, 2004, Vol.313, pages 453 to 458 | 5-12 |
| A | LU Zhenhui, Participation of autophagy in the degeneration process of rat hepatocytes after transplantation following prolonged cold preservation, Arch.Histol.Cytol., 2005, Vol.68, No.1, pages 71 to 80 | 5-12 |
| P,X | WO 2005/40095 A1 (ASTRAZENECA AB), 06 May, 2005 (06.05.05), Example 31<br>& GB 324236 A | 1,4 |
| P,X | WO 2005/49006 A1 (Ajinomoto Co., Inc.), 02 June, 2005 (02.06.05), Claim 1<br>(Family: none) | 1,3-4 |

Form PCT/ISA/210 (continuation of second sheet) (April 2005)

**INTERNATIONAL SEARCH REPORT**

International application No.
PCT/JP2006/308844

Continuation of A. CLASSIFICATION OF SUBJECT MATTER
(International Patent Classification (IPC))

***A61P25/28***(2006.01), ***A61P29/00***(2006.01), ***61P35/00***(2006.01), ***A61P43/00***(2006.01), ***C12N9/99***(2006.01), ***G01N33/15***(2006.01), ***G01N33/50***(2006.01)

(According to International Patent Classification (IPC) or to both national classification and IPC)

Form PCT/ISA/210 (extra sheet) (April 2005)

**INTERNATIONAL SEARCH REPORT**

International application No.
PCT/JP2006/308844

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

A special technical feature of the invention according to claims 1-4 relates to a compound represented by the general formula (1) or (2) having a Dpp4 inhibitory action, and a special technical feature of the invention according to claims 5-14 mainly relates to an autophagy regulator and a method for screening drugs utilizing Dpp4 function using Dpp4 antibody.

There is no technical relationship among these inventions involving one or more of the same or corresponding special technical features, therefore, they cannot be recognized as being so linked as to form a single general inventive concept.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003171271 A


### Non-patent literature cited in the description


- *Science,* 2004, vol. 306, 990-995
- *Biochem. Biophys. Res. Com.,* 2004, vol. 313, 453-458
- *Ann. Rev. Nutr.,* 1987, vol. 7, 539-564
- *J. Biol. Chem.,* 1994, vol. 269, 25348-25353
- *Biochem. Biophys. Res. Com.,* 2004, vol. 313, 387-458
- *Neurosci Lett.,* 2004, vol. 354, 166-168
- *Clinical Science,* 2000, vol. 99, 93-104
- *Biochem. J.,* 2003, vol. 371, 525-532
- *Adv. Exp. Med. Biol.,* 1997, vol. 421, 171-178
- *Diabetes,* 2004, vol. 53, 2181-2189
- *Diabetes Care,* 2003, vol. 26, 2929-2940
- *Physiology & Behavior,* 2003, vol. 80, 123-134
- Preparation of isolated liver cells. **SEGLEN, P.O.** Methods in Cell Biology. Academic, 1976, vol. 13, 29-83
- **VENERANDO, R. ; MIOTTO, G. ; KADOWAKI, M. ; SILIPRANDI, N. ; MORTIMORE, G.E.** Multiphasic control of proteolysis by leucine and alanine in the isolated rat hepatocytes. *Am. J. Physiol.,* 1994, vol. 266, C455-C461
- **TAPUHI, Y ; SCHMIDT, D. ; LINDNER, W. ; KARGER, B.L.** Dansylation of amino acids for high-performance liquid chromatography. *Anal. Biochem.,* 1981, vol. 115, 123-129
- *Journal of Neurochemistry,* 2002, vol. 82, 538-549
- *Arch Histol Cytol,* 2005, vol. 68 (1), 71-80